(19) Europäisches Patentamt
European Patent Office
Office européen des brevets

(11) EP 1 273 585 B1

(12) **EUROPEAN PATENT SPECIFICATION**

(45) Date of publication and mention of the grant of the patent:
**16.02.2005 Bulletin 2005/07**

(51) Int Cl.[7]: **C07D 493/04**

(21) Application number: **02012340.2**

(22) Date of filing: **05.06.2002**

(54) **Chiral compounds**

Chirale Verbindungen

Composées chirales

(84) Designated Contracting States:
**AT BE CH CY DE DK ES FI FR GB GR IE IT LI LU MC NL PT SE TR**

(30) Priority: **02.07.2001 EP 01114896**

(43) Date of publication of application:
**08.01.2003 Bulletin 2003/02**

(73) Proprietor: **MERCK PATENT GmbH**
**64293 Darmstadt (DE)**

(72) Inventors:
- **May, Alison Linda**
  **Corfe Muller, Dorset BH21 3XF (GB)**
- **Greenfield, Simon, Dr.**
  **Wimborne, Dorset BH21 1SY (GB)**
- **Goulding, Mark John, Dr.**
  **Ringwood, Hampshire BH24 1SH (GB)**
- **Parri, Owain Llyr, Dr.**
  **Poole, Dorset BH15 2LN (GB)**

(56) References cited:
**WO-A-95/16007** **WO-A-98/00428**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 273 585 B1

Printed by Jouve, 75001 PARIS (FR)

## Description

### Field of the Invention

The invention relates to chiral compounds with a high helical twisting power, to liquid crystal mixtures comprising them, and to chiral linear or crosslinked liquid crystal polymers obtained from a polymerizable mixture comprising one or more chiral compounds. The invention further relates to the use of the chiral compounds and the mixtures and polymers obtained thereof in liquid crystal displays, active and passive optical elements, adhesives, synthetic resins with anisotropic mechanical properties, cosmetic and pharmaceutical compositions, diagnostics, liquid crystal pigments, for decorative and security applications, nonlinear optics, optical information storage or as chiral dopants.

### Background and Prior Art

For many applications it is desirable to have LC mixtures with a twisted phase. Among these are e.g. phase-change displays, guest-host displays, passive and active matrix TN and STN displays like AMD-TN, ferroelectric displays and cholesteric displays like SSCT (surface stabilized cholesteric texture) or PSCT (polymer stabilized cholesteric texture) displays, including displays with temperature compensated characteristics, e.g. by appropriate selection of the cholesteric compounds according to the invention either alone or in combination with further chiral dopants. For these applications it is advantageous to have available a chiral dopant with a high HTP in order to reduce the amount of dopant needed to induce the desired pitch.

For some applications it is desired to have LC mixtures that exhibit a strong helical twist and thereby a short pitch length. For example in liquid crystal mixtures that are used in selectively reflecting cholesteric displays like SSCT or PSCT, the pitch has to be selected such that the maximum of the wavelength reflected by the cholesteric helix is in the range of visible light. Another possible application are polymer films with a chiral liquid crystal phase for optical elements, such as cholesteric broadband polarizers or retardation films.

In a cholesteric LC material, the pitch p of the molecular helix in the first approximation, which is sufficient for most practical applications, is inversely proportional to the concentration c of the chiral dopant in the liquid crystal host mixture according to equation (1):

$$p = \frac{1}{HTP} \cdot \frac{1}{C} \qquad (1)$$

The proportionality factor is the helical twisting power (HTP) of the chiral dopant.

As can be seen from equation (1), a short pitch can be achieved by using high amounts of dopant or by using a dopant with a high HTP. However, the chiral dopants of prior art often exhibit low values of the HTP, so that high amounts of dopant are needed. This is a disadvantage because chiral dopants can be used only as pure enantiomers and are therefore expensive and difficult to synthesize.

Furthermore, when using chiral dopants of prior art in high amounts, they often negatively affect the properties of the liquid crystal host mixture, such as e.g. the clearing point, the dielectric anisotropy $\Delta\varepsilon$, the viscosity, the driving voltage or the switching times.

Also, the chiral dopants often show a high temperature dependence of the helical twisting power. When used in cholesteric mixtures this leads to a strong temperature dependence of the reflection wavelength of the mixture. This is especially disadvantageous for application in cholesteric displays, such as SSCT displays, where usually a low temperature dependence of the reflection wavelength is required.

Another disadvantage of prior art chiral compounds is that they often show low solubility in the liquid crystal host mixture, which leads to undesired crystallization at low temperatures. To overcome this disadvantage, typically two or more different chiral dopants have to be added to the host mixture. This implies higher costs and also requires additional effort for temperature compensation of the mixture, as the different dopants have to be selected such that their temperature coefficients of the twist compensate each other.

Consequently, there is a considerable demand for chiral compounds with a high HTP which are easy to synthesize, can be used in low amounts, show low temperature dependence of the twisting power e.g. for utilizing a constant reflection wavelength, do not affect the properties of the liquid crystal host mixture and show good solubility in the host mixture.

The invention has the aim of providing chiral compounds having these properties, but which do not have the disadvantages of the chiral dopants of the state of the art as discussed above. Another aim of the invention is to extend the pool of chiral dopants available to the expert.

It has been found that these aims can be achieved by providing chiral isosorbide derivatives as described below.

WO 95/16007 and WO 98/00428 disclose chiral mesogenic compounds with a high HTP based on 1,4:3,6-Dianhydro-D-sorbitol (isosorbide). However, many of these compounds do only have a limited solubility in liquid crystal host mixtures.

The inventors have now found that chiral compounds derived from isosorbide

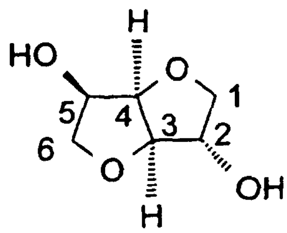

that is substituted unsymmetrically with two different mesogenic groups in 2- and 5-position show a higher solubility in liquid crystal host mixtures compared to isosorbide derivatives that are substituted symmetrically with two identical mesogenic grops. Furthermore, it was found that the unsymmetrically substituted isosorbides show a remarkable difference in solubility depending on the respective position of the mesogenic groups. For example, an isosorbide derivative with a larger mesogenic group, like a two-ring group, attached in 5-position and a smaller mesogenic group, like a one-ring group, attached in 2-position has a considerably higher solubility than its corresponding isomer where the two-ring group is attached in 2-position and the one-ring group in 5-position. This is a considerable improvement which could not be expected from prior art.

<u>Summary of the Invention</u>

One object of the present invention are chiral compounds of formula I

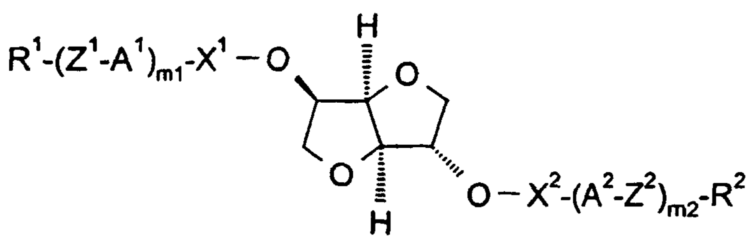

I

wherein

| | |
|---|---|
| $R^1$ and $R^2$ | are independently of each other F, Cl, Br, I, CN, SCN, $SF_5$, straight chain or branched alkyl with up to 30 C atoms which may be unsubstituted, mono- or poly-substituted by F, Cl, Br, I or CN, it being also possible for one or more non-adjacent $CH_2$ groups to be replaced, in each case independently from one another, by -O-, -S-, -NH-, -$NR^0$-, -CO-, -COO-, -OCO-, -OCO-O-, -S-CO-, -CO-S-, -CH=CH-or -C≡C- in such a manner that O and/or S atoms are not linked directly to one another, or P-Sp-X, |
| R° | is H or alkyl with 1 to 4 C atoms, |
| P | is a polymerizable group, |
| Sp | is a spacer group or a single bond, |
| X | is -O-, -S-, -$OCH_2$-, -$CH_2O$-, -CO-, -COO-, -OCO-, -OCO-O-, -CO-$NR^0$-, -$NR^0$-CO-, -$OCH_2$-, -$CH_2O$-, -$SCH_2$-, -$CH_2S$-, -CH=CH-COO-, -OOC-CH=CH- or a single bond, |
| $X^1$ | is -CO-, -OCO-, -$NR^0$-CO-, -CH=CH-CO-, -$CH_2$-, -$C_2H_4$-, -$CF_2$- or a single bond, |

$X^2$ is -CO-, -COO-, -CO-NR$^0$-, -CO-CH=CH-, -$CH_2$-, -$C_2H_4$-, -$CF_2$- or a single bond,

$Z^1$ and $Z^2$ are independently of each other -O-, -S-, -CO-, -COO-,-OCO-, -O-COO-, -CO-NR$^0$-, -NR$^0$-CO-, -$OCH_2$-, -$CH_2O$-, -$SCH_2$-, -$CH_2S$-, -$CF_2O$-, -$OCF_2$-, -$CF_2S$-, -$SCF_2$-,-$CH_2CH_2$-, -$CF_2CH_2$-, -$CH_2CF_2$-, -$CF_2CF_2$-, -CH=N-,-N=CH-, -N=N-, -CH=CH-, -CF=CH-, -CH=CF-, -CF=CF-, -C≡C-, -CH=CH-COO-, -OCO-CH=CH- or a single bond,

$A^1$ and $A^2$ are independently of each other an aliphatic or aromatic carbocyclic or heterocyclic group with up to 16 C atoms that may also comprise fused rings and may be unsubstituted, mono- or polysubstituted with L,

L is halogen or a cyano, nitro, alkyl, alkoxy, alkylcarbonyl or alkoxycarbonyl group with 1 to 7 C atoms, wherein one or more H atoms may be substituted by F or Cl,

m1 is 1, 2 or 3, and

m2 is 0, 1, 2 or 3,

with the proviso that the total number of fused or unfused rings in the group $(Z^1\text{-}A^1)_{m1}$ is larger than in the group $(A^2\text{-}Z^2)_{m2}$.

Another object of the invention is a liquid crystal mixture containing at least one compound of formula I.

Another object of the present invention is a polymerizable liquid crystal mixture comprising at least one compound of formula I.

Another object of the invention is a linear or crosslinked anisotropic polymer with twisted structure obtainable from a polymerizable liquid crystal mixture comprising one or more compounds of formula I.

A further object of the invention is the use of compounds of formula I or a liquid crystal mixture or anisotropic polymer film comprising them in liquid crystal displays, such as STN, TN, AMD-TN, temperature compensation, ferroelectric, guest-host, phase change or surface stabilized or polymer stabilized cholesteric texture (SSCT, PSCT) displays, in active and passive optical elements like polarizers, compensators, alignment layers, colour filters or holographic elements, in adhesives, synthetic resins with anisotropic mechanical properties, cosmetic and pharmaceutical compositions, diagnostics, liquid crystal pigments, for decorative and security applications, in nonlinear optics, optical information storage or as chiral dopants.

Yet another object of the invention is a liquid crystal display comprising a liquid crystal mixture comprising at least one chiral compound of formula I.

## Detailed Description of the Invention

The inventive chiral compounds bear several advantages

- they exhibit a high HTP,
- they exhibit a good solubility in liquid crystal mixtures,
- when inventive compounds are used as chiral dopant in a liquid crystal mixture, due to their high solubility higher amounts of dopant can be used to produce a high twist (= a low pitch),
- due to their high HTP, lower amounts of inventive dopants are needed to achieve a high pitch, and thereby the liquid crystalline properties of the mixture are less negatively affected,
- enantiomerically pure inventive chiral compounds are easy to prepare.

The inventive chiral compounds are mesogenic or even liquid crystalline, i.e. they can induce or enhance mesophase behaviour for example in admixture with other compounds, or even exhibit one or more mesophases themselves. It is also possible that the inventive compounds show mesophase behaviour only in mixtures with other compounds, or, in case of polymerizable compounds, when being (co)polymerized. Mesogenic inventive chiral compounds are especially preferred.

In formula I, $Z^1$ adjacent to $R^1$ and $Z^2$ adjacent to $R^2$ are preferably a single bond.

Preferred groups $A^1$ and $A^2$ in formula I are for example cyclopentane, 1,1,-dimethylcyclopentane, tetrahydrofuran, pyrrolidine, furan, pyrrol, thiophene, oxazole, thiazole, thiadiazole, imidazole, pyrrolidinone, cyclohexane,

cyclohexene, tetrahydropyran, piperidine, tetrahydrothiopyrane, dioxane, dithiane, oxathiane, thiomorpholine, morpholine, phenylene, pyridine, pyrimidine, pyrazine, bicyclohexane, bicyclohexene, cyclohexane-1,4-dione, bicyclo[2,2,2]octylene, cyclohexenone, indane, naphthalene, decahydronaphthalene, 1,2,3,4-tetrahydronaphthalene, anthracene and phenanthrene.

Particularly preferably $A^1$ and $A^2$ are selected from 1,4-phenylene in which, in addition, one or more CH groups may be replaced by N, 1,4-cyclohexylene in which, in addition, one or two non-adjacent $CH_2$ groups may be replaced by O and/or S, 1,3-dioxolane-4,5-diyl, 1,4-cyclohexenylene, 1,4-bicyclo-(2,2,2)-octylene, piperidine-1,4-diyl, naphthalene-2,6-diyl, decahydronaphthalene-2,6-diyl, 1,2,3,4-tetrahydronaphthalene-2,6-diyl and indane-2,5-diyl, it being possible for all these groups to be unsubstituted, mono- or polysubstituted by L as defined above.

Especially preferred are compounds of formula I wherein the groups $(Z^2\text{-}A^1)_{m1}$ and $(A^2\text{-}Z^2)_{m2}$ contain only monocyclic groups $A^1$ and $A^2$, with m1 > m2.

Of these preferred compounds, especially preferred are those wherein $A^1$ and $A^2$ are selected from aliphatic and aromatic six-membered carbocycles that may also contain one or more hetero atoms, very preferably from 1,4-phenylene in which, in addition, one or more CH groups may be replaced by N, 1,4-cyclohexylene in which, in addition, one or two non-adjacent $CH_2$ groups may be replaced by O and/or S, 1,3-dioxolane-4,5-diyl, 1,4-cyclohexenylene and piperidine-1,4-diyl, it being possible for all these groups to be unsubstituted, mono- or polysubstituted with L as defined in formula I.

A smaller group of preferred groups $(Z^1\text{-}A^1)_{m1}$ and $(A^2\text{-}Z^2)_{m2}$ is listed below. For reasons of simplicity, Phe in these groups is 1,4-phenylene which may also substituted by at least one group L as defined in formula I, and Cyc is 1,4-cyclohexylene. The following list of preferred groups is also comprising their mirror images

-Phe- II-1

-Cyc- II-2

-Phe-Z-Phe- II-3

-Phe-Z-Cyc- II-4

-Cyc-Z-Cyc- II-5

-Phe-Z-Phe-Z-Phe- II-6

-Phe-Z-Phe-Z-Cyc- II-7

-Phe-Z-Cyc-Z-Phe- II-8

-Cyc-Z-Phe-Z-Cyc- II-9

-Cyc-Z-Cyc-Z-Phe- II-10

-Cyc-Z-Cyc-Z-Cyc- II-11

Especially preferred are formulae II-1, I-2, II-3, II-4 and II-5. Further preferred are groups $(Z^1\text{-}A^1)_{m1}$ and $(A^2\text{-}Z^2)_{m2}$ comprising at least one group Phe that is substituted with one or two groups L, preferably in 2- and/or 3-position, and L is F, Cl, $CH_3$, $OCH_3$, $CF_3$, $CHF_2$, $CH_2F$, $OCF_3$ $OCHF_2$, $OCH_2F$ or CN.

Further preferred are compounds of formula I, wherein

- m1 is 1 and m2 is 0 or 1.
- m1 is 2 and m2 is 1.
- $X^1$ and $X^2$ are -CO-.
- $Z^1$ and $Z^2$ are independently of each other -COO-, -OCO- or a single bond.
- $R^1$ and/or $R^2$ is a polymerizable group P-Sp-X.
- $R^1$ and $R^2$ are straight chain or branched alkyl or alkoxy with 1 to 12 C atoms.
- L is F, Cl, CN, $NO_2$, $CH_3$, $C_2H_5$, $OCH_3$, $OC_2H_5$, $COCH_3$, $COC_2H_5$, $CF_3$, $CHF_2$, $CH_2F$, $OCF_3$ $OCHF_2$, $OCH_2F$ or $OC_2F_5$.
- $A^1$ and $A^2$ are selected from trans-1,4-cyclohexylene and 1,4-phenylene, the latter being unsubstituted or substituted with up to 4, preferably 1 or 2 groups L, preferably in 3- and/or 5-position or in 2- and/or 3-position, with L being preferably F, Cl, $CH_3$, $OCH_3$, $CF_3$, $CHF_2$, $CH_2F$, $OCF_3$ $OCHF_2$, $OCH_2F$ or CN.
- $A^1$ and $A^2$ are unsubstituted 1,4-phenylene.

L is preferably F, Cl, CN, $NO_2$, $CH_3$, $C_2H_5$, $OCH_3$, $OC_2H_5$, $COCH_3$, $COC_2H_5$, $CF_3$, $CHF_2$, $CH_2F$, $OCF_3$ $OCHF_2$, $OCH_2F$, $OC_2F_5$, in particular F, Cl, CN, $CH_3$, $CHF_2$, $C_2H_5$, $OCH_3$, $OCHF_2$, $CF_3$ and $OCF_3$, most preferably F, $CH_3$, $CF_3$, $OCH_3$, $OCHF_2$ and $OCF_3$.

If $R^1$ or $R^2$ in formula I is an alkyl or alkoxy radical, i.e. where the terminal $CH_2$ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one $CH_2$ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Halogen is preferably F or Cl.

$R^1$ or $R^2$ in formula I can be a polar or a non-polar group. In case of a polar group, it is selected from CN, $SF_5$, halogen, $OCH_3$, SCN, $COR^5$, $COOR^5$ or a mono- oligo- or polyfluorinated alkyl or alkoxy group with 1 to 4 C atoms. $R^5$ is optionally fluorinated alkyl with 1 to 4, preferably 1 to 3 C atoms. Especially preferred polar groups are selected of F, Cl, CN, $OCH_3$, $COCH_3$, $COC_2H_5$, $COOCH_3$, $COOC_2H_5$, $CF_3$, $CHF_2$, $CH_2F$, $OCF_3$, $OCHF_2$, $OCH_2F$, $C_2F_5$ and $OC_2F_5$, in particular F, Cl, CN, $CF_3$, $OCHF_2$ and $OCF_3$. In case of a non-polar group, it is preferably alkyl with up to 15 C atoms or alkoxy with 2 to 15 C atoms.

$R^1$ or $R^2$ in formula I can be an achiral or a chiral group. In case of a chiral group it is preferably selected of formula III:

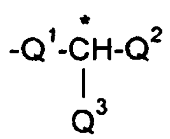

III

wherein

$Q^1$ is an alkylene or alkylene-oxy group with 1 to 9 C atoms or a single bond,

$Q^2$ is an alkyl or alkoxy group with 1 to 10 C atoms which may be unsubstituted, mono- or polysubstituted by F, Cl, Br or CN, it being also possible for one or more non-adjacent $CH_2$ groups to be replaced, in each case independently from one another, by -C≡C-, -O-, -S-, -NH-, $-N(CH_3)$, -CO-, -COO-, -OCO-, -OCO-O-, -S-CO- or -CO-S- in such a manner that oxygen atoms are not linked directly to one another,

$Q^3$ is F, Cl, Br, CN or an alkyl or alkoxy group as defined for $Q^2$ but being different from $Q^2$.

In case $Q^1$ in formula III is an alkylene-oxy group, the O atom is preferably adjacent to the chiral C atom.

Preferred chiral groups of formula III are 2-alkyl, 2-alkoxy, 2-methylalkyl, 2-methylalkoxy, 2-fluoroalkyl, 2-fluoroalkoxy, 2-(2-ethin)-alkyl, 2-(2-ethin)-alkoxy, 1,1,1-trifluoro-2-alkyl and 1,1,1-trifluoro-2-alkoxy.

Particularly preferred chiral groups are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy,

2-ethylhexoxy, 1-methylhexoxy, 2-octyloxy, 2-oxa-3-methylbutyl, 3-oxa-4-methylpentyl, 4-methylhexyl, 2-hexyl, 2-octyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy.

In addition, compounds of formula I containing an achiral branched group $R^1$ or $R^2$ may occasionally be of importance, for example, due to a reduction in the tendency towards crystallization. Branched groups of this type generally do not contain more than one chain branch. Preferred achiral branched groups are isopropyl, isobutyl (=methylpropyl), isopentyl (=3-methylbutyl), isopropoxy, 2-methylpropoxy and 3-methylbutoxy.

The polymerisable group P is preferably selected from $CH_2=CW^1-COO-$,

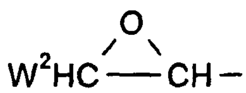 , 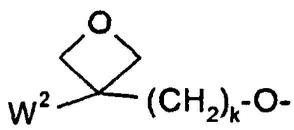 ,

$CH_2=CW^2-(O)_{k1}-$, $CH_3-CH=CH-O-$, $HO-CW^2W^3-$, $HS-CW^2W^3-$, $HW^2N-$, $HO-CW^2W^3-NH-$, $CH_2=CW^1-CO-NH-$, $CH_2=CH-(COO)_{k1}-Phe-(O)_{k2}-$, $Phe-CH=CH-$, HOOC-, OCN- and $W^4W^5W^6Si-$, with $W^1$ being H, Cl, CN, phenyl or alkyl with 1 to 5 C-atoms, in particular H, Cl or $CH_3$, $W^2$ and $W^3$ being independently of each other H or alkyl with 1 to 5 C-atoms, in particular methyl, ethyl or n-propyl, $W^4$, $W^5$ and $W^6$ being independently of each other Cl, oxaalkyl or oxacarbonylalkyl with 1 to 5 C-atoms, Phe being 1,4-phenylene and $k_1$ and $k_2$ being independently of each other 0 or 1.

Especially preferably P is a vinyl group, an acrylate group, a methacrylate group, a propenyl ether group or an epoxy group, especially preferably an acrylate or a methacrylate group.

As for the spacer group Sp all groups can be used that are known for this purpose to the skilled in the art. The spacer group Sp is preferably a linear or branched alkylene group having 1 to 20 C atoms, in particular 1 to 12 C atoms, in which, in addition, one or more non-adjacent $CH_2$ groups may be replaced by -O-, -S-, -NH-,$-NR^0-$, $-SiR^0R^{00}-$, -CO-, -COO-, -OCO-, -OCO-O-, -S-CO-, -CO-S-,CH=CH- or $-C\equiv C-$ in such a manner that O and/or S atoms are not linked directly to one another, wherein $R^0$ and $R^{00}$ are independently of each other H or alkyl with 1 to 4 C-atoms.

Typical spacer groups are for example $-(CH_2)_p-$, $-(SiR^0R^{00}-O)_p-$, $-(CH_2CH_2O)_r-CH_2CH_2-$, $-CH_2CH_2-S-CH_2CH_2-$ or $-CH_2CH_2-NH-CH_2CH_2-$, with p being an integer from 2 to 12 and r being an integer from 1 to 3.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylenethioethylene, ethylene-N-methyliminoethylene, 1-methylalkylene, ethenylene, propenylene and butenylene for example.

Especially preferred are inventive chiral compounds of formula I wherein Sp-X is denoting alkylene or alkyleneoxy with 2 to 6 C atoms. Straight-chain groups are especially preferred.

In another preferred embodiment of the invention the chiral compounds comprise at least one spacer group Sp that is a chiral group of formula IV:

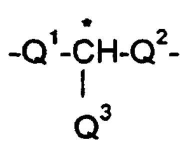

IV

wherein

- $Q^1$ is an alkylene or alkylene-oxy group with 1 to 10 C atoms or a single bond,
- $Q^2$ is an alkylene or alkylene-oxy group with 1 to 10 C atoms or a single bond, being different from $Q^1$, and
- $Q^3$ is halogen, a cyano group or an alkyl or alkoxy group with 1 to 4 C atoms different from $Q^2$.

In case $Q^1$ in formula IV is an alkylene-oxy group, the O atom is preferably adjacent to the chiral C atom.

Particularly preferred compounds of formula I are those of the following formulae

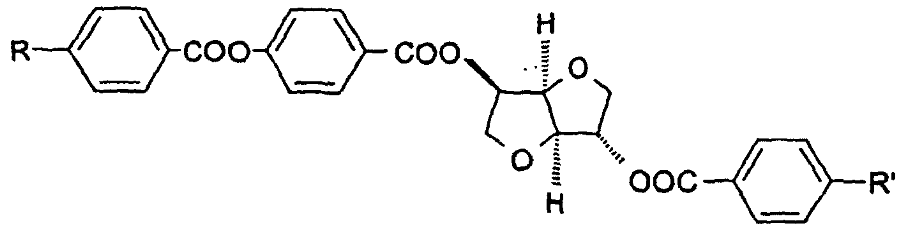

I-1

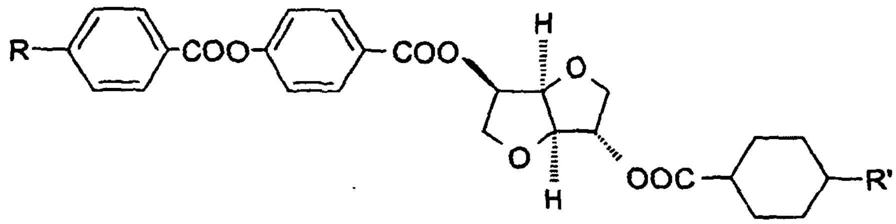

I-2

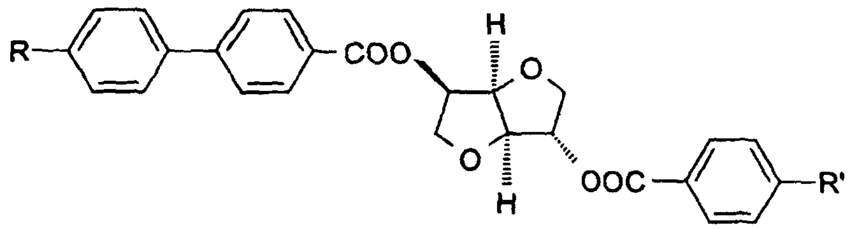

I-3

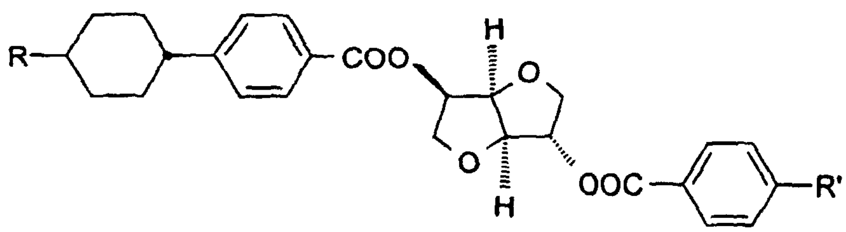

I-4

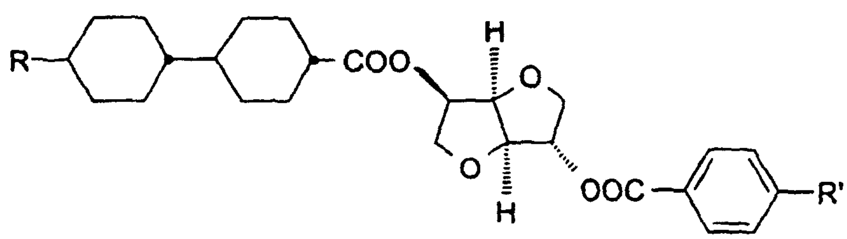

I-5

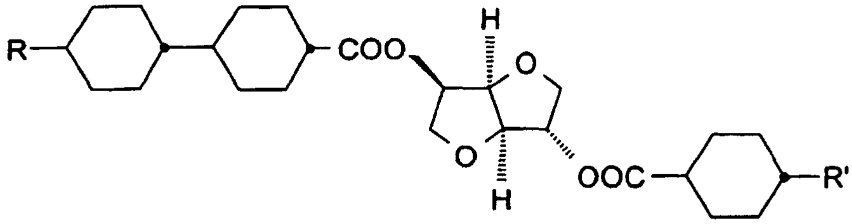

I-6

Wherein R and R' have one of the meanings of $R^1$ in formula I and the phenylene rings also may be substituted by 1, 2 or 3 groups L as defined above.

In these preferred compounds R and R' are preferably alkyl or alkoxy with 1 to 12 C atoms or P-Sp-X- as defined above. In case of compounds with substituted phenylene rings these are preferably substituted in 2-, 3- 2- and 3- or 3- and 5-position with F,Cl, $CH_3$, $OCH_3$ or CN.

The inventive chiral compounds can be synthesized according to or in analogy to known methods as described for example in WO 98/00428.

The preparation of unsymmetrical isosorbides of formula I is possible due to a difference in reactivity between the two hydroxy groups on the isosorbide. Thus, the 2-position in the isosorbide is least hindered and therefore reacts preferentially. Selective esterification of 1,4:3,6-Dianhydro-D-glucitol is described in Z. Cekovic and Z. Tokic, Communications, Synthesis, p. 610-612 (1989). It is possible to utilize this method to prepare e.g. monoesters of isosorbide and then to react the other hydroxy groups with a different acid.

Further methods of synthesis can be taken from the examples.

The chiral compounds of formula I can be used in a liquid crystal mixture for displays exhibiting a helically twisted molecular structure of the liquid crystal matrix like, for example, TN displays of the active or passive matrix type, STN, phase-change, guest-host, ferroelectric or cholesteric displays like SSCT (surface stabilized cholesteric texture) or PSCT (polymer stabilized cholesteric texture).

Thus, another object of the invention is a liquid crystal mixture, in particular a chiral smectic or cholesteric liquid crystal mixture, comprising at least one chiral compound of formula I.

Yet another object of the invention is a liquid crystal display comprising a liquid crystal medium containing at least one chiral compound of formula I.

The chiral compounds of formula I are characterized by high values of the HTP. This enables the preparation of liquid crystal mixtures with a high helical twist, i.e. a low pitch, by using only low amounts of chiral compounds of formula I. This is a considerable advantage, as it is often observed that the addition of high amounts of chiral dopants to a liquid crystal mixture negatively affects its liquid crystal phase behaviour and electrooptical properties, such as the dielectric anisotropy, the viscosity or the clearing point. Thus, by using chiral compounds of formula I in a liquid crystal mixture or display its properties are altered only to a minor extent, compared to prior art dopants, resulting for example in a lower threshold voltage and faster switching times of the display.

The chiral compounds of formula I are further characterized by a high solubility in a liquid crystal host mixture. Undesired spontaneous crystallization at low temperatures is reduced, and the operating temperature range of the mixture can be broadened. The use of a second dopant, which is often added to avoid crystallization, can thus be avoided.

A particularly preferred embodiment of the present invention therefore relates to a liquid crystal mixture comprising only one chiral compound, which is a compound of formula I, and to a display comprising such a mixture.

The chiral compounds of formula I also show a low temperature dependence of the HTP when added to a liquid crystal host mixture. They are thus useful as chiral dopants for liquid crystal mixtures and displays with a low temperature dependence of the pitch.

A liquid crystal mixture according to the invention comprises preferably 0.1 to 30 %, in particular 1 to 25 % and very particularly preferably 2 to 15 % by weight of chiral compounds of formula I. It preferably comprises 1, 2 or 3 chiral compounds of formula I.

The compounds of formula I are especially suitable for use in cholesteric liquid crystal mixtures for cholesteric displays, in particular SSCT or PSCT displays. Cholesteric displays are described for example in WO 92/19695, WO 93/23496, US 5,453,863 or US 5,493,430, the entire disclosure of these documents being introduced into this application by way of reference.

It was found that, when using chiral compounds of formula I as dopants in liquid crystal mixtures for cholesteric displays, due to their high solubility and low temperature dependence of the HTP a mixture with high helical twist and

low temperature dependence can be achieved. Cholesteric mixtures with high brightness and low temperature dependence of the reflection colour can thus be achieved even by using only one chiral dopant according to formula I. This is a considerable advantage over prior art, where it is often necessary to use two or more dopants to prevent crystallisation, and to use two dopants with opposite temperature dependence of the helical twist (e.g. one with positive temperature dependence and one with negative temperature dependence) to achieve good temperature compensation of the reflection wavelength.

Thus, a particularly preferred embodiment of the present invention relates to a cholesteric liquid crystal medium, in particular for use in SSCT and PSCT displays, comprising one chiral dopant, which is a compound of formula I, preferably in an amount of 1 to 20 %, in particular 2 to 15 %.

For the applications described above the liquid crystal mixture preferably contains a chiral component which contains at least one chiral compound of formula I, and a nematic component comprising one or more nematic or nematogenic compounds.

Preferably the liquid crystal mixture consists of 2 to 25, preferably 3 to 15 compounds, at least one of which is a chiral compound of formula I. The other compounds, forming the nematic component, are preferably low molecular weight liquid crystal compounds selected from nematic or nematogenic substances, for example from the known classes of the azoxybenzenes, benzylidene-anilines, biphenyls, terphenyls, phenyl or cyclohexyl benzoates, phenyl or cyclohexyl esters of cyclohexanecarboxylic acid, phenyl or cyclohexyl esters of cyclohexylbenzoic acid, phenyl or cyclohexyl esters of cyclohexylcyclohexanecarboxylic acid, cyclohexylphenyl esters of benzoic acid, of cyclohexanecarboxylic acid and of cyclohexylcyclohexanecarboxylic acid, phenylcyclohexanes, cyclohexylbiphenyls, phenylcyclohexylcyclohexanes, cyclohexylcyclohexanes, cyclohexylcyclohexenes, cyclohexylcyclohexylcyclohexenes, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexylpyridazines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2-(4-phenylcyclohexyl)-ethanes, 1-cyclohexyl-2-biphenyl-ethanes, 1-phenyl2-cyclohexylphenylethanes, optionally halogenated stilbenes, benzyl phenyl ether, tolanes, substituted cinnamic acids and further classes of nematic or nematogenic substances. The 1,4-phenylene groups in these compounds may also be laterally mono- or difluorinated.

The liquid crystal mixture of this preferred embodiment is based on the achiral compounds of this type.

The most important compounds that are possible as components of these liquid crystal mixtures can be characterized by the following formula

R'-L'-G'-E-R"

wherein L' and E, which may be identical or different, are in each case, independently from one another, a bivalent radical from the group formed by -Phe-, -Cyc-, -Phe-Phe-, -Phe-Cyc-, -Cyc-Cyc-, -Pyr-, -Dio-, -B-Phe- and -B-Cyc- and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl abd B is 2-(trans-1,4-cyclohexyl)ethyl, pyrimidine-2,5-diyl, pyridine-2,5-diyl or 1,3-dioxane-2,5-diyl.

G' in these compounds is selected from the following bivalent groups -CH=CH-, -N(O)N-, -CH=CY-, -CH=N(O)-, -C≡C-, $-CH_2-CH_2-$, -CO-O-, $-CH_2-O-$, -CO-S-, $-CH_2-S-$, -CH=N-, -COO-Phe-COO- or a single bond, with Y being halogen, preferably chlorine, or -CN.

R' and R" are, in each case, independently of one another, alkyl, alkenyl, alkoxy, alkenyloxy, alkanoyloxy, alkoxycarbonyl or alkoxycarbonyloxy with 1 to 18, preferably 3 to 12 C atoms, or alternatively one of R' and R" is F, $CF_3$, $OCF_3$, Cl, NCS or CN.

In most of these compounds R' and R" are, in each case, independently of each another, alkyl, alkenyl or alkoxy with different chain length, wherein the sum of C atoms in nematic media generally is between 2 and 9, preferably between 2 and 7.

Many of these compounds or mixtures thereof are commercially available. All of these compounds are either known or can be prepared by methods which are known per se, as described in the literature (for example in the standard works such as Houben-Weyl, Methoden der Organischen Chemie [Methods of Organic Chemistry], Georg-Thieme-Verlag, Stuttgart), to be precise under reaction conditions which are known and suitable for said reactions. Use may also be made here of variants which are known per se, but are not mentioned here.

Polymerizable compounds of formula I or polymerizable liquid crystal mixtures comprising one or more compounds of formula I are useful for the preparation of polymerizable mixtures, which can be used for example in polymer stabilized liquid crystal displays, such as PSCT (polymer stabilized cholesteric texture) and anisotropic polymer gels, which can be used for example in scattering type displays. Anisotropic polymer gels and displays comprising them are disclosed for example in DE 195 04 224 and GB 2 279 659.

The chiral compounds of formula I and polymerizable liquid crystal mixtures comprising them are particularly

useful for the preparation of anisotropic polymer films with helically twisted molecular structure with uniform planar orientation, i.e. wherein the helical axis is oriented perpendicular to the plane of the film.

For example, oriented cholesteric polymer films can be used as broad waveband reflective polarizers like for example described in EP 0 606 940, WO 97/35219 or EP 0 982 605, as colour filters, for security markings, or for the preparation of liquid crystal pigments for decorative or security uses.

For the preparation of anisotropic polymer gels or oriented polymer films, the liquid crystal mixture should comprise at least one polymerizable compound, which can be a compound of formula I or an additional polymerizable mesogenic or liquid crystalline compound.

Thus, another object of the invention are polymerizable liquid crystal mixtures comprising at least one chiral compound of formula I.

Examples of suitable polymerizable mesogenic compounds that can be used as components of the polymerizable liquid crystal mixture, are disclosed for example in WO 93/22397; EP 0,261,712; DE 195,04,224; WO 95/22586 and WO 97/00600. The compounds disclosed in these documents, however, are to be regarded merely as examples that shall not limit the scope of this invention.

Preferably the polymerizable liquid crystal mixture comprises at least one polymerizable mesogenic compound having one polymerizable functional group and at least one polymerizable mesogenic compound having two or more polymerizable functional groups.

Examples of especially useful monoreactive chiral and achiral polymerizable mesogenic compounds are shown in the following list of compounds, which should, however, be taken only as illustrative and is in no way intended to restrict, but instead to explain the present invention:

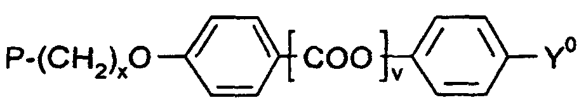

(Va)

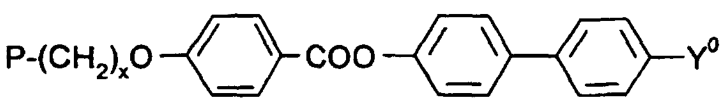

(Vb)

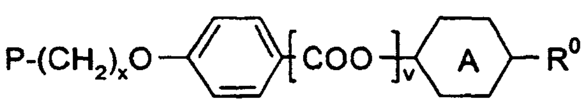

(Vc)

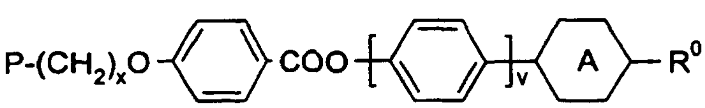

(Vd)

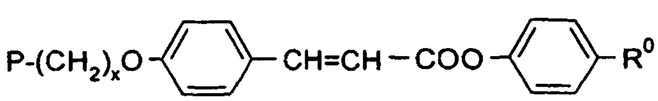

(Ve)

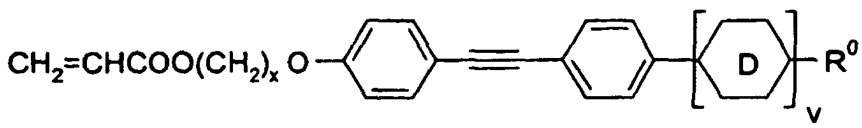

(Vf)

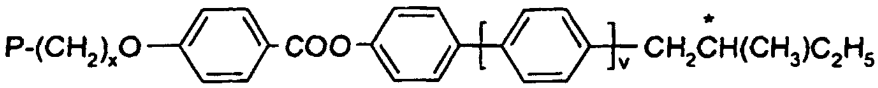

(Vg)

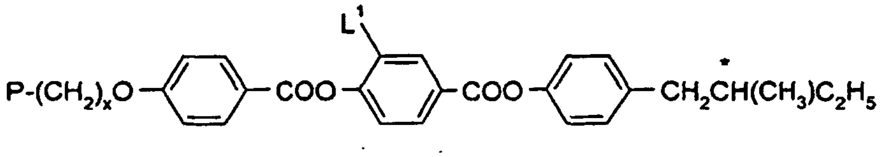

(Vh)

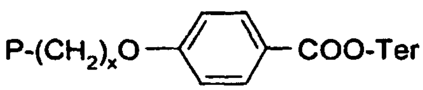

(Vi)

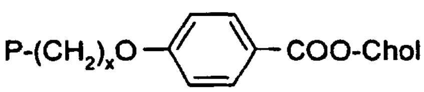

(Vk)

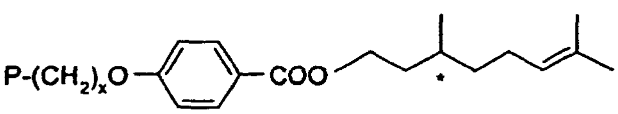

(Vm)

wherein, P has one of the meanings given above, x is an integer from 1 to 12 , A and D are 1,4-phenylene or 1,4-cyclohexylene, v is 0 or 1, $Y^0$ is a polar group, $R^0$ is a non-polar alkyl or alkoxy group, Ter is a terpenoid radical like e.g. menthyl, Chol is a cholesteryl group, and $L^1$ and $L^2$ are each independently H, F, Cl, CN, OH, $NO_2$ or an optionally halogenated alkyl, alkoxy or tarbonyl group with 1 to 7 C atoms.

The polar group $Y^0$ is preferably CN, $NO_2$, halogen, $OCH_3$, OCN, SCN, $COR^5$, $COOR^5$ or a mono- oligo- or polyfluorinated alkyl or alkoxy group with 1 to 4 C atoms. $R^5$ is optionally fluorinated alkyl with 1 to 4, preferably 1 to 3 C atoms. Especially preferably the polar group $Y^0$ is selected of F, Cl, CN, $NO_2$, $OCH_3$, $COCH_3$, $COC_2H_5$, $COOCH_3$, $COOC_2H_5$, $CF_3$, $C_2F_5$, $OCF_3$, $OCHF_2$, and $OC_2F_5$, in particular F, Cl, CN, $OCH_3$ and $OCF_3$.

The non-polar group $R^0$ is preferably an alkyl group with 1 or more, preferably 1 to 15 C atoms or an alkoxy group with 2 or more, preferably 2 to 15 C atoms.

Examples of useful direactive chiral and achiral polymerizable mesogenic compounds are shown in the following list of compounds, which should, however, be taken only as illustrative and is in no way intended to restrict, but instead to explain the present invention

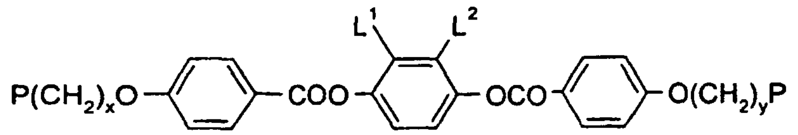

(VIa)

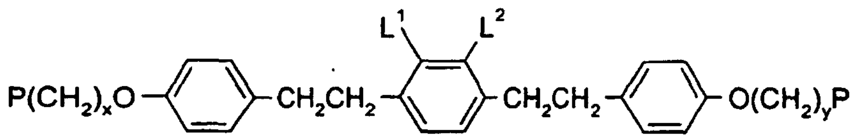

(VIb)

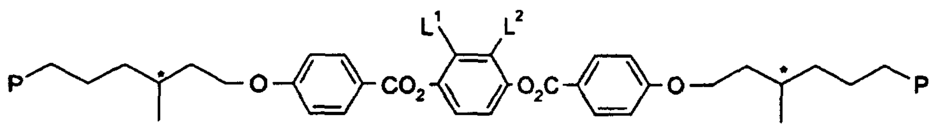

(VIc)

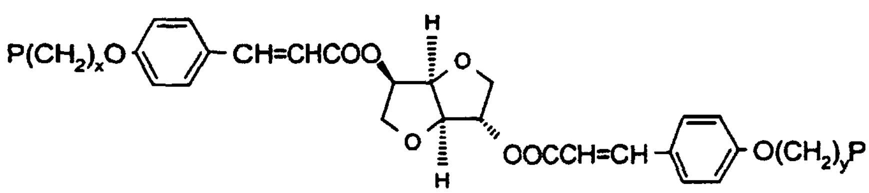

(VId)

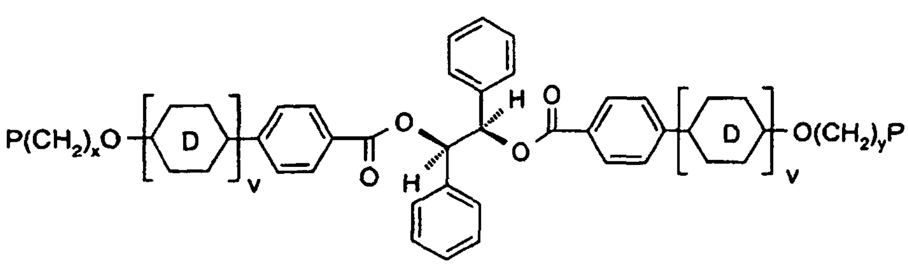

(VIe)

wherein P, x, D, $L^1$ and $L^2$ have one of the meanings given above and y is an integer from 1 to 12 the same as or different from x.

A polymerizable liquid crystal material according to the first preferred embodiment as described above comprises one or more chiral dopants which themselves do not necessarily have to show a liquid crystal phase and give good planar alignment themselves, in particular non-polymerizable chiral dopants.

The mono- and difunctional polymerizable mesogenic compounds of above formulae V and VI can be prepared by methods which are known per se and which are described in the documents cited above and, for example, in standard works of organic chemistry such as, for example, Houben-Weyl, Methoden der organischen Chemie, Thieme-Verlag, Stuttgart.

In a preferred embodiment of the invention the polymerizable liquid crystal mixtures comprise at least one inventive chiral compound, at least one monofunctional compound of formulae Va-Vm and at least one bifunctional polymerizable compound of formulae VIa-VIe.

In another preferred embodiment the polymerizable liquid crystal mixtures comprise at least one inventive

chiral compound and at least two monofunctional compounds of formulae Va-Vm.

Another object of the invention is an anisotropic polymer film with an oriented chiral liquid crystalline phase obtainable by (co)polymerizing a liquid crystal mixture comprising at least one chiral compound of formula I and at least one polymerizable mesogenic compound preferably selected of formula Va-Vm and VIa-VIe and/or at least one polymerizable chiral compound of formula I.

To prepare anisotropic polymer film with a chiral liquid crystalline phase with uniform orientation the polymerizable liquid crystal can be coated onto a substrate, aligned and polymerized in situ, for example by exposure to heat or actinic radiation, to fix the uniform orientation of the liquid crystal molecules. Alignment and curing are carried out in the liquid crystalline phase of the mixture.

Actinic radiation means irradiation with light, like UV light, IR light or visible light, irradiation with X-rays or gamma rays or irradiation with high energy particles, such as ions or electrons. As a source for actinic radiation for example a single UV lamp or a set of UV lamps can be used. Another possible source for actinic radiation is a laser, like e.g. a UV laser, an IR laser or a visible laser.

For example, when polymerizing by means of UV light, a photoinitiator can be used that decomposes under UV irradiation to produce free radicals or ions that start the polymerization reaction.

It is also possible to use a cationic photoinitiator, when curing reactive mesogens with for example vinyl and epoxide reactive groups, that photocures with cations instead of free radicals.

As a photoinitiator for radical polymerization for example the commercially available Irgacure 651, Irgacure 184, Darocure 1173 or Darocure 4205 (all from Ciba Geigy AG) can be used, whereas in case of cationic photopolymerization the commercially available UVI 6974 (Union Carbide) can be used.

Preferably the polymerizable liquid crystal mixture comprising polymerizable chiral compounds of formula I and/or polymerizable mesogenic compounds of formulae V and VI additionally comprises 0.01 to 10 %, in particular 0.05 to 8 %, very preferably 0.1 to 5 % by weight of a photoinitiator, especially preferably a UV-photoinitiator.

Polymerization is preferably carried out under an atmosphere of inert gas, preferably under a nitrogen atmosphere.

As a substrate for example a glass or quarz sheet as well as a plastic film or sheet can be used. It is also possible to put a second substrate on top of the coated mixture prior to, during and/or after polymerization. The substrates can be removed after polymerization or not. When using two substrates in case of curing by actinic radiation, at least one substrate has to be transmissive for the actinic radiation used for the polymerization.

Isotropic or birefringent substrates can be used. In case the substrate is not removed from the polymerized film after polymerization, preferably isotropic substrates are used.

Preferably at least one substrate is a plastic substrate such as for example a film of polyester such as polyethyleneterephthalate (PET), of polyvinylalcohol (PVA), polycarbonate (PC) or triacetylcellulose (TAC), especially preferably a PET film or a TAC film. As a birefringent substrate for example an uniaxially stretched plastic film can be used. For example PET films are commercially available from ICI Corp. under the trade name Melinex.

Preferably the polymerizable liquid crystal mixture I is coated as a thin layer on a substrate or between substrate, and aligned in its chiral mesophase into planar orientation, wherein the axis of the molecular helix extends transversely to the layer.

Planar orientation can be achieved for example by shearing the mixture, e.g. by means of a doctor blade. It is also possible to put a second substrate on top of the coated material. In this case, the shearing caused by putting together the two substrates is sufficient to give good alignment. Alternatively it is possible to apply an alignment layer, for example a layer of rubbed polyimide or sputtered $SiO_x$, on top of at least one of the substrates, or to apply an electric or magnetic field to the coated mixture, in order to induce or enhance planar alignment. In a preferred method planar alignment is induced or enhanced by addition of one or more surface-active compounds to the polymerizable mixture.

In some cases it is of advantage to apply a second substrate not only to aid alignment of the polymerizable mixture but also to exclude oxygen that may inhibit the polymerization. Alternatively the curing can be carried out under an atmosphere of inert gas. However, curing in air is also possible using suitable photoinitiators and high lamp power. When using a cationic photoinitiator oxygen exclusion most often is not needed, but water should be excluded.

A detailed description of the in situ polymerization of polymerizable mesogenic compounds can be found in D.J.Broer et al., Makromolekulare Chemie 190, 2255 (1989).

A polymerizable liquid crystal mixture for the preparation of anisotropic polymer films comprises preferably 0.1 to 35 %, in particular 0.5 to 15 % and very particularly preferably 0.5 to 5 % by weight of one or more polymerizable chiral compounds of formula I.

Polymerizable liquid crystal mixtures are preferred that comprise 1 to 3 chiral compounds of formula I.

The inventive polymerizable liquid crystal mixtures can additionally comprise one or more other suitable components, such as, for example, catalysts, sensitizers, stabilizers, co-reacting monomers or surface-active compounds.

In a preferred embodiment of the invention, the inventive polymerizable liquid crystal mixture comprises a stabilizer that is used to prevent undesired spontaneous polymerization for example during storage of the composition.

As stabilizers in principal all compounds can be used that are known to the skilled in the art for this purpose. These compounds are commercially available in a broad variety. Typical examples for stabilizers are 4-ethoxyphenol or butylated hydroxytoluene (BHT).

It is also possible, in order to increase crosslinking of the polymers, to add up to 20% of a non mesogenic compound with two or more polymerizable functional groups to the polymerizable composition alternatively or additionally to the multifunctional polymerizable mesogenic compounds.

Typical examples for difunctional non mesogenic monomers are alkyldiacrylates or alkyldimethacrylates with alkyl groups of 1 to 20 C atoms. Typical examples for non mesogenic monomers with more than two polymerizable groups are trimethylpropanetrimethacrylate or pentaerythritoltetraacrylate.

Polymerization of inventive compositions comprising compounds with only one polymerizable functional group leads to linear polymers, whereas in the presence of compounds with more than one polymerizable functional group crosslinked polymers are obtained.

For the preparation of anisotropic polymer gels, the liquid crystal mixtures can be polymerized in situ as described above, however, in this case alignment of the polymerizable mixture is not necessary.

The inventive chiral compounds of formula I and liquid crystal mixtures, liquid crystal polymers or liquid crystal pigments comprising them are also suitable for use in cosmetic and pharmaceutical compositions, for example for coloured make-up as described in EP 815 826 or as UV-filters for the protection of human skin or hair, in particular protection against UV-A and UV-B-radiation, as described for example in DE 196 29 761 or EP 1 038 941. The inventive dopants have a high HTP, therefore only small amounts are needed to yield a short pitch, resulting in a material that shows reflection in the UV range and is suitable as UV-filter.

A liquid crystal mixture, liquid crystal polymer or liquid crystal pigment comprising a chiral compound of formula I and reflecting UV light, in particular of a wavelength of 200 to 400 nm, is another object of the invention. Another object is a cosmetic composition, in particular a cosmetic or pharmaceutical composition for protection of human skin or hair, comprising as UV-filter a liquid crystal mixture, liquid crystal polymer or liquid crystal pigment comprising a chiral compound of formula I and reflecting UV light, in particular in a wavelength range of 200-440 nm, especially 280-400 nm, 200-230 nm (UV-C) and 280-330 nm (UV-B).

From the foregoing description, one skilled in the art can easily ascertain the essential characteristics of this invention, and without departing from the spirit and scope thereof, can make various changes and modifications of the invention to adapt it to various usages and conditions.

Without further elaboration, it is believed that one skilled in the art can, using the preceding description, utilize the present invention to its fullest extent. The following examples are, therefore, to be construed as merely illustrative and not limitative of the remainder of the disclosure in any way whatsoever.

In the foregoing and in the following examples, unless otherwise indicated, all temperatures are set forth uncorrected in degrees Celsius and all parts and percentages are by weight.

The values of the helical twisting power HTP of a chiral compound in a liquid crystal host are given according to the equation HTP = $(p*c)^{-1}$ in $\mu m^{-1}$, wherein p is the pitch of the molecular helix, given in $\mu m$, and c is the concentration by weight of the chiral compound in the host given in relative values (thus, e.g. a concentration of 1 % by weight is corresponding to a value of c of 0.01).

The following abbreviations are used to illustrate the liquid crystalline phase behaviour of the compounds: C = crystalline; N = nematic; S = smectic; N*, Ch = chiral nematic or cholesteric; I = isotropic. The numbers between these symbols indicate the phase transition temperatures in degree Celsius. Furthermore, mp. is the melting point, $\Delta n$ is the birefringence at 589 nm and 20 °C and $\Delta\varepsilon$ is the dielectric anisotropy at 20 °C. C* in a chemical formula denotes a chiral C atom. DCC is N,N'-Dicyclohexylcarbodiimide, DMAP is 4-Dimethylaminopyridine, DCM is dichloromethane. "Conventional workup" means: water is added if necessary, the mixure is extracted with methylene chloride, diethyl ether or toluene, the phases are separated, the organic phase is dried and concentrated by evaporation, and the product is purified by crystallization and/or chromatography.

Unless indicated otherwise, the HTP values of the examples were determined in the commercially available liquid crystal host mixture BL087 (Merck KGaA, Darmstadt, Germany) at a concentration of 5 % and a temperature of 20 °C.

Example 1

Compound (1) is prepared according to the following reaction scheme

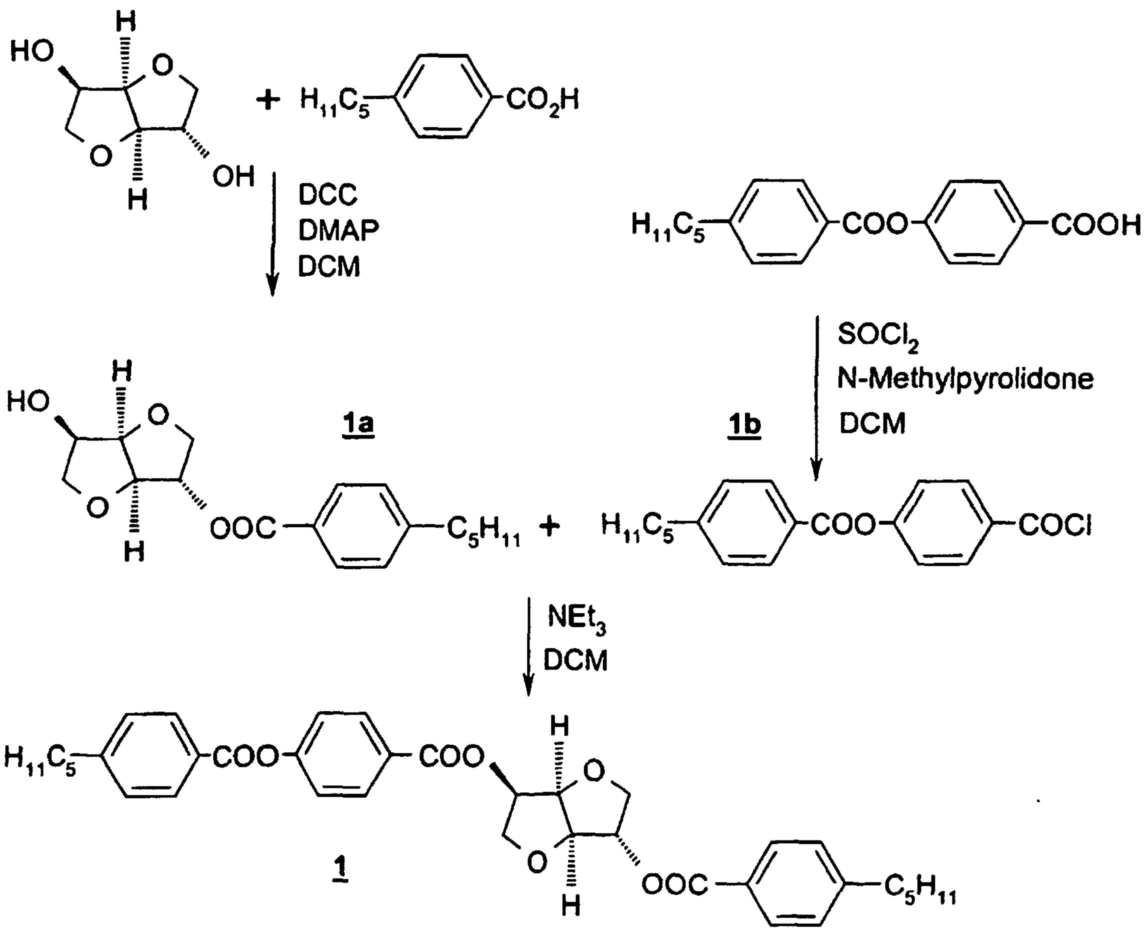


1a

1b

1

Preparation of monoester (compound 1a)

Isosorbide (3.8g, 0.026mol), 4-n-pentylbenzoic acid (5.0g, 0.026mol) and N,N'-dicyclohexylcarbodiimide (5.36g, 0.052mol) were combined in dichloromethane (100ml). 4-Dimethylaminopyridine (0.1g, catalyst) was added and the reaction mixture was stirred at room temperature overnight with the exclusion of moisture ($CaCl_2$ guard tube). N,N-Dicyclohexylurea was filtered off and the filtrate was washed with water (3x100ml), dried with anhydrous $Na_2SO_4$ and the solvent removed under reduced pressure using a rotary evaporator. The crude product was flash column chromatographed over MERCK 40-63 μm silica gel, eluting with 3:2 toluene: ethyl acetate; 4.11g (49.5%) yield.

Preparation of acid chloride (compound 1b)

The two ring acid (1.55g, 0.00496mol) and thionyl chloride (0.64g, 0.00546mol) were combined in dichloromethane (20ml). N-Methylpyrollidone was added and the reaction mixture was stirred and refluxed until a clear solution was obtained and then for a further 2 hours. Reaction mixture was evaporated to dryness under reduced pressure using a rotary evaporator. The crude product was used in the next step without further purification.

Preparation of unsymmetrical diester (compound 1)

Monoester (1.44g, 0.00451 mol), triethylamine (2.28g, 0.0025mol) and dichloromethane (20ml) were combined with stirring at 25°C. Acid chloride (1.64g, 0.00496mol) in dichloromethane (20ml) was added dropwise and the reaction mixture was stirred for 24 hours in a nitrogen atmosphere. The reaction mixture was then poured into water and extracted with dichloromethane (50ml). The organic extract was washed with water, dried with anhydrous $Na_2SO_4$ and the solvent was removed under reduced pressure using a rotary evaporator. The crude product was flash columned over MERCK 40-63µm silica gel, eluting with dichloromethane. The columned material was recrystallised from ethanol and dried in a vacuum oven at 40°C for 24 hours; 0.92g (33.2%) yield.

The following compounds can be prepared analoguously

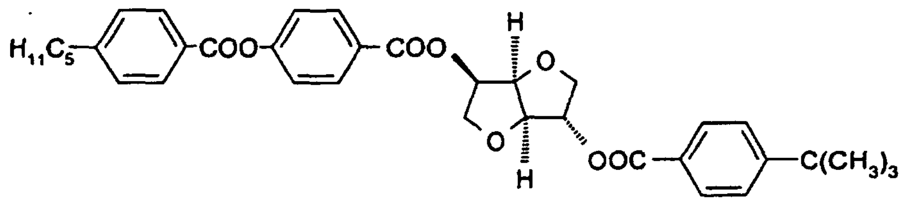

2

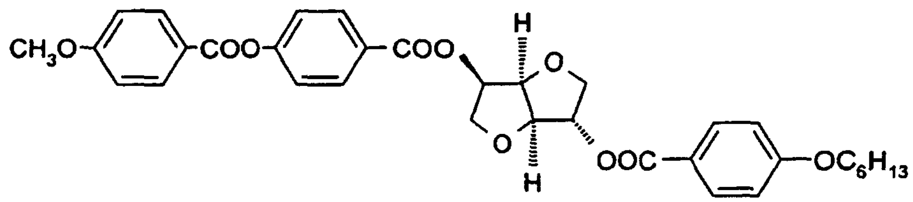

3

The corresponding isomers 1*, 2* and 3* of compounds 1-3, where the two-ring is at the 2-position of the isosorbide core, were prepared analoguously using the two ring acid in the first step.

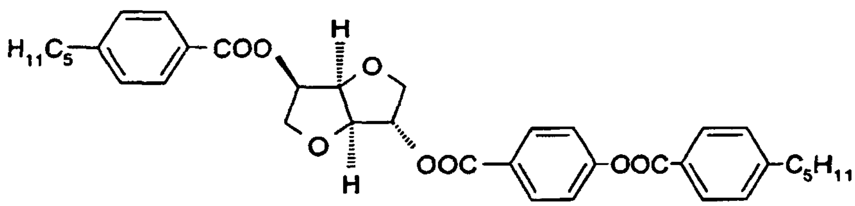

1*

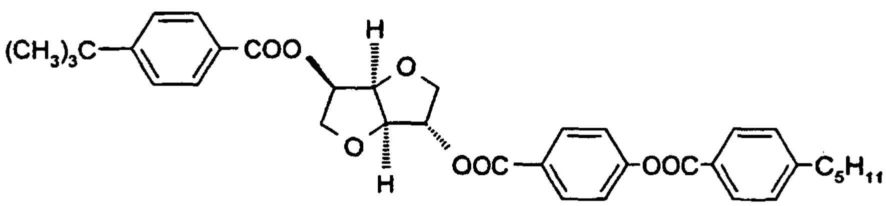

2*

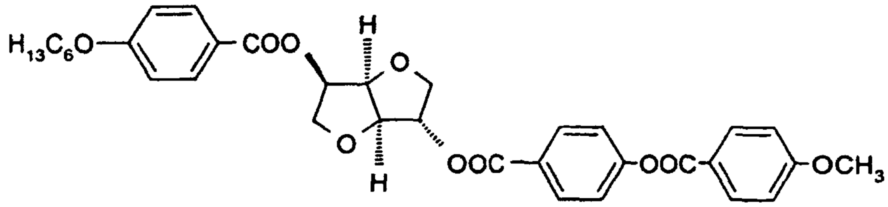

3*

The HTP and temperature dependence of the reflection wavelength in the temperature range from -20 °C to +80 °C of compounds 1-3 and their isomers 1*-3* were measured in the nematic host mixture BL087 at a concentration of 5 %. The solubility of compounds 1-3and their isomers 1*-3* was measured by preparing a mixture of the respective compound in BL087 at a concentration of 20 %. The mixtures were allowed to stand at room temperature for 1 week to allow for crystallistion. Then the mixtures were filtered, the reflection wavelength measured and the concentration of the chiral compound remaining in the mixture calculated from the known HTP of the compounds. The results are shown in table 1.

Table 1:

| Comparison of compounds 1-3 and their isomers 1*-3* | | | | |
|---|---|---|---|---|
| No. | mp. [°C] | HTP [$\mu m^{-1}$] | d$\lambda$/dT [nm/°C] | Solubility at room temp. |
| 1 | 101.6 | 63.7 | 0.18 | 15.3% |
| 1* | 116.4 | 60.4 | 0.16 | 4.2% |
| 2 | 112.8 | 69.8 | 0.73 | 11.8% |
| 2* | 140.4 | 50.8 | 0.46 | 2.3% |
| 3 | 121.8 | 67.4 | 0.09 | 4.1% |
| 3* | 129.8 | 62.1 | 0.32 | 0.8% |

Compounds 1-3 according to the present invention have higher HTP and considerably higher solubility in the nematic host mixture than their isomers 1*-3*.

## Claims

**1.** Chiral compounds of formula I

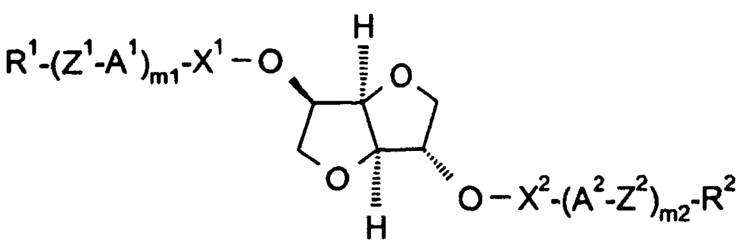

I

wherein

$R^1$ and $R^2$ are independently of each other F, Cl, Br, I, CN, SCN, $SF_5$, straight chain or branched alkyl with up to 30 C atoms which may be unsubstituted, mono- or poly-substituted by F, Cl, Br, I or CN, it being also possible for one or more non-adjacent $CH_2$ groups to be replaced, in each case independently from one another, by -O-, -S-, -NH-, -$NR^0$-, -CO-, -COO-, -OCO, -OCO-O-, -S-CO-, -CO-S-, -CH=CH- or -C≡C- in such a manner that O and/or S atoms are not linked directly to one another, or P-Sp-X,

$R^0$ is H or alkyl with 1 to 4 C atoms,

P is a polymerizable group,

Sp is a spacer group or a single bond,

X is -O-, -S-, $-OCH_2-$, $-CH_2O-$, -CO-, -COO-, -OCO-, -OCO-O-, $-CO-NR^0-$, $-NR^0-CO-$, $-OCH_2-$, $-CH_2O-$, $-SCH_2-$, $-CH_2S-$, -CH=CH-COO-, -OOC-CH=CH- or a single bond,

$X^1$ is -CO-, -OCO-, $-NR^0-CO-$, -CH=CH-CO-, $-CH_2-$,$-C_2H_4-$, $-CF_2-$ or a single bond,

$X^2$ is -CO-, -COO-, $-CO-NR^0-$, -CO-CH=CH-, $-CH_2-$,$-C_2H_4-$, $-CF_2-$ or a single bond,

$Z^1$ and $Z^2$ are independently of each other -O-, -S-, -CO-,-COO-, -OCO-, -O-COO-, $-CO-NR^0-$, $-NR^\circ-CO-$, $-OCH_2-$, $-CH_2O-$, $-SCH_2-$, $-CH_2S-$, $-CF_2O-$, $-OCF_2-$,$-CF_2S-$, $-SCF_2-$ $-CH_2CH_2-$, $-CF_2CH_2-$, $-CH_2CF_2-$, $-CF_2CF_2-$, -CH=N-, -N=CH-, -N=N-, -CH=CH-,-CF=CH-, -CH=CF-, -CF=CF-, -C≡C-, -CH=CH-COO-, -OCO-CH=CH- or a single bond,

$A^1$ and $A^2$ are independently of each other an aliphatic or aromatic carbocyclic or heterocyclic group with up to 16 C atoms that may also comprise fused rings and may be unsubstituted, mono- or polysubstituted with L,

L is halogen or a cyano, nitro, alkyl, alkoxy, alkylcarbonyl or alkoxycarbonyl group with 1 to 7 C atoms, wherein one or more H atoms may be substituted by F or Cl,

m1 is 1, 2 or 3, and

m2 is 0, 1, 2 or 3,

with the proviso that the total number of fused or unfused rings in the group $(Z^1-A^1)_{m1}$ is larger than in the group $(A^2-Z^2)_{m2}$.

**2.** Chiral compounds according to claim 1, wherein $A^1$ and $A^2$ are selected from 1,4-phenylene in which, in addition, one or more CH groups may be replaced by N, 1,4-cyclohexylene in which, in addition, one or two non-adjacent $CH_2$ groups may be replaced by O and/or S, 1,3-dioxolane-4,5-diyl, 1,4-cyclohexenylene and piperidine-1,4-diyl, it being possible for all these groups to be unsubstituted, mono- or polysubstituted with L as defined in formula I, and wherein m1 > m2.

**3.** Chiral compounds according to claim 1 or 2, wherein m1 is 2 and m2 is 1.

**4.** Chiral compounds according to at least one of claims 1 to 3, wherein $X^1$ and $X^2$ are -CO-.

**5.** Chiral compounds according to at least one of claims 1 to 4, wherein $A^1$ and $A^2$ are selected from trans-1,4-cyclohexylene and 1,4-phenylene, the latter being unsubstituted or substituted with up to 4 groups L.

**6.** Chiral compounds according to at least one of claims 1 to 5, wherein $Z^1$ and $Z^2$ are -COO-, -OCO or a single bond.

**7.** Chiral compounds according to at least one of claims 1 to 6, wherein $R^1$ and/or $R^2$ is a polymerizable group P-Sp-X.

**8.** Chiral compounds according to at least one of claims 1 to 7, selected from the following formulae

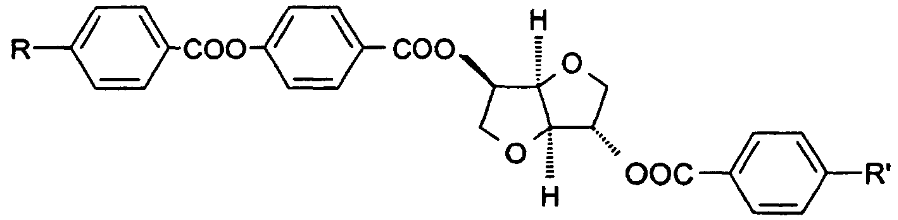

I-1

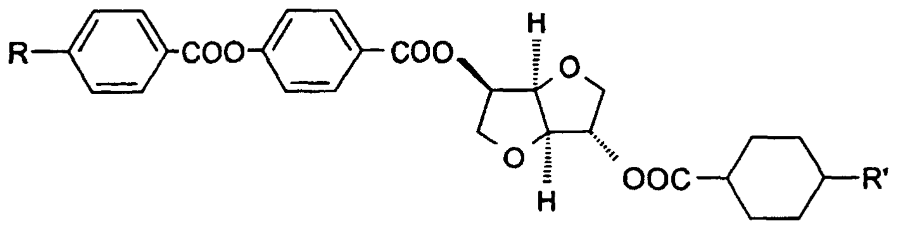

I-2

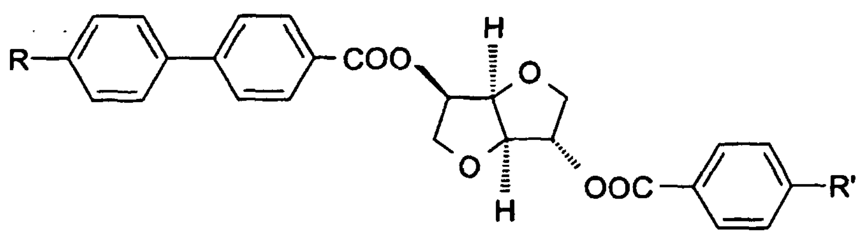

I-3

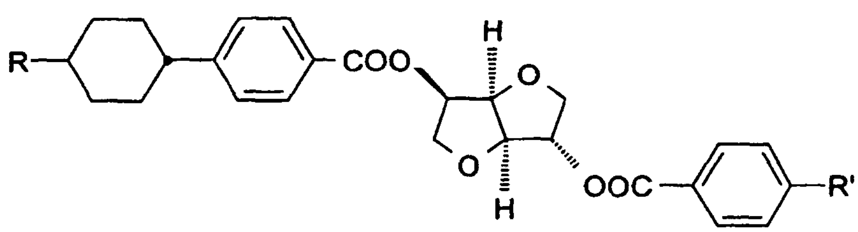

I-4

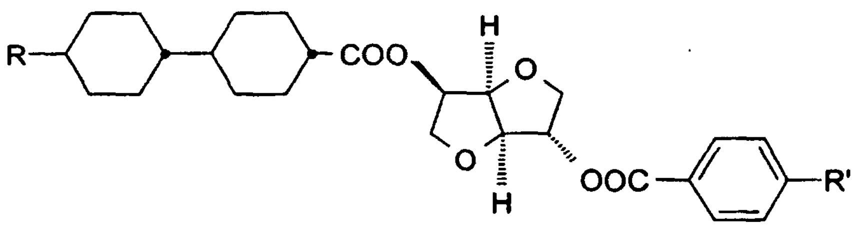

I-5

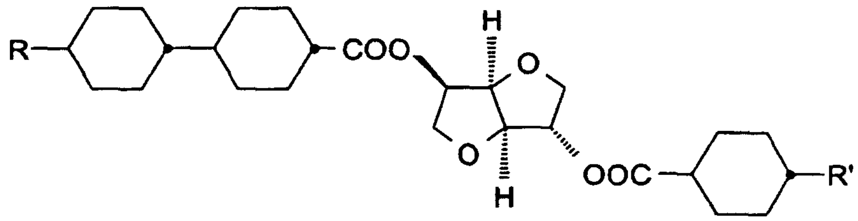

I-6

wherein R and R' have one of the meanings of $R^1$ in claim 1 and the phenylene rings also may be substituted by 1, 2 or 3 groups L as defined in claim 1.

**9.** A liquid crystal mixture comprising at least one chiral compound according to at least one of claims 1 to 8.

**10.** A polymerizable liquid crystal mixture comprising at least one compound of formula I according to at least one of claims 1 to 8 and at least one polymerizable mesogenic compound, which can be said compound of formula I and/or an additional polymerizable mesogenic compound.

**11.** A chiral linear or crosslinked liquid crystal polymer obtainable by polymerizing a mixture according to claim 10.

**12.** Use of a chiral compound, liquid crystal mixture or polymer according to at least one of claims 1 to 10 in liquid crystal displays, like for example STN, TN, AMD-TN, temperature compensation, ferroelectric, guest-host, phase change or surface stabilized or polymer stabilized cholesteric texture (SSCT, PSCT) displays, in active and passive optical elements like polarizers, compensators, alignment layers, colour filters or holographic elements, in adhesives, synthetic resins with anisotropic mechanical properties, cosmetic or pharmaceutical compositions for example as UV filters, diagnostics, liquid crystal pigments, for decorative and security applications, in nonlinear optics, optical information storage or as chiral dopants.

**13.** A liquid crystal display comprising a liquid crystal mixture according to claim 9 or 10.

**14.** A cholesteric liquid crystal mixture comprising a chiral component which contains at least one chiral compound according to at least one of claims 1 to 8, and further comprising a nematic component which contains one or more nematic or nematogenic compounds.

**15.** A cholesteric or SSCT display comprising a mixture according to claim 14.

**16.** A colour filter, broadband reflective polarizer, patterned film or security marking obtained from a polymerizable mixture according to claim 10.

**Patentansprüche**

**1.** Chirale Verbindungen der Formel I

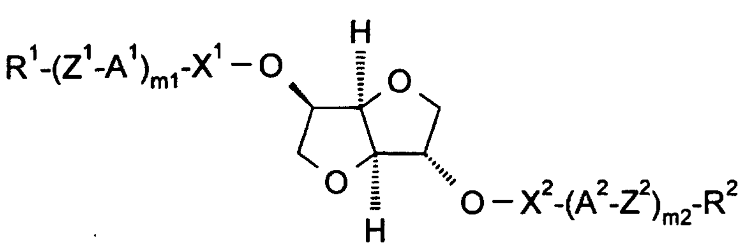

I

worin

$R^1$ und $R^2$ unabhängig voneinander F, Cl, Br, I, CN, SCN, $SF_5$, geradkettiges oder verzweigtes Alkyl mit bis zu 30 C-Atomen, das unsubstituiert oder ein- oder mehrfach durch F, Cl, Br, I oder CN substituiert sein kann, wobei auch eine oder mehrere nicht benachbarte $CH_2$-Gruppen jeweils unabhängig voneinander durch-O-, -S-, -NH-, -$NR^0$-, -CO-, -COO-, -OCO-, -OCO-O-,-S-CO-, -CO-S-, -CH=CH- oder -C≡C- so ersetzt sein können, dass O- und/oder S-Atome nicht direkt miteinander verknüpft sind, oder P-Sp-X bedeuten,

$R^0$ H oder Alkyl mit 1 bis 4 C-Atomen bedeutet,

P eine polymerisierbare Gruppe bedeutet,

Sp eine Spacergruppe oder eine Einfachbindung bedeutet,

X -O-, -S-, -$OCH_2$-, -$CH_2O$-, -CO-, -COO-, -OCO-, -OCO-O-, -CO-$NR^0$-, -$NR^0$-CO-, -$OCH_2$-, -$CH_2O$-, -$SCH_2$-, -$CH_2S$-, -CH=CH-COO-, -OOC-CH=CH- oder eine Einfachbindung bedeutet,

$X^1$ -CO-, -OCO-, -$NR^0$-CO-, -CH=CH-CO-, -$CH_2$-, -$C_2H_4$-, -$CF_2$- oder eine Einfachbindung bedeutet,

$x^2$ -CO-, -COO-, -CO-$NR^0$-, -CO-CH=CH-, -$CH_2$-, -$C_2H_4$-, -$CF_2$- oder eine Einfachbindung bedeutet,

$Z^1$ und $Z^2$ unabhängig voneinander -O-, -S-, -CO-, -COO-, -OCO-, -O-COO-, -CO-$NR^0$-, -$NR^0$-CO-, -$OCH_2$-, -$CH_2O$-, -$SCH_2$-, -$CH_2S$-, -$CF_2O$-, -$OCF_2$-, -$CF_2S$-, -$SCF_2$-, -$CH_2CH_2$-, -$CF_2CH_2$-, -$CH_2CF_2$-, -$CF_2CF_2$-, -CH=N-, -N=CH-, -N=N-, -CH=CH-, -CF=CH-, -CH=CF-, -CF=CF-, -C≡C-, -CH=CH-COO-, -OCO-CH=CH- oder eine Einfachbindung bedeuten,

$A^1$ und $A^2$ unabhängig voneinander eine aliphatische oder aromatische carbocyclische oder heterocyclische Gruppe mit bis zu 16 C-Atomen bedeuten, die auch anellierte Ringe enthalten und unsubstituiert oder ein-oder mehrfach mit L substituiert sein kann,

L Halogen oder eine Cyan-, Nitro-, Alkyl-, Alkoxy-, Alkylcarbonyl- oder Alkoxycarbonylgruppe mit 1 bis 7 C-Atomen bedeutet, worin ein oder mehrere H-Atome durch F oder Cl substituiert sein können,

m1 1, 2 oder 3 bedeutet und

m2 0, 1, 2 oder 3 bedeutet,

mit der Maßgabe, dass die Gesamtzahl anellierter oder nicht anellierter Ringe in der Gruppe $(Z^1\text{-}A^1)_{m1}$ größer als in der Gruppe $(A^2\text{-}Z^2)_{m2}$ ist.

2. Chirale Verbindungen nach Anspruch 1, worin $A^1$ und $A^2$ ausgewählt sind aus 1,4-Phenylen, in dem zusätzlich eine oder mehrere CH-Gruppen durch N ersetzt sein können, 1,4-Cyclohexylen, in dem zusätzlich eine oder zwei nicht benachbarte $CH_2$-Gruppen durch O und/oder S ersetzt sein können, 1,3-Dioxolan-4,5-diyl, 1,4-Cyclohexenylen und Piperidin-1,4-diyl, wobei alle diese Gruppen unsubstituiert oder ein- oder mehrfach mit L wie in Formel I definiert substituiert sein können, und worin m1 > m2.

3. Chirale Verbindungen nach Anspruch 1 oder 2, worin m1 2 und m2 1 ist.

4. Chirale Verbindungen nach mindestens einem der Ansprüche 1 bis 3, worin $X^1$ und $X^2$ -CO- bedeuten.

5. Chirale Verbindungen nach mindestens einem der Ansprüche 1 bis 4, worin $A^1$ und $A^2$ ausgewählt sind aus trans-1,4-Cyclohexylen und 1,4-Phenylen, wobei Letzteres unsubstituiert oder mit bis zu 4 Gruppen L substituiert ist.

6. Chirale Verbindungen nach mindestens einem der Ansprüche 1 bis 5, worin $Z^1$ und $Z^2$ -COO-, -OCO oder eine Einfachbindung bedeuten.

7. Chirale Verbindungen nach mindestens einem der Ansprüche 1 bis 6, worin $R^1$ und/oder $R^2$ eine polymerisierbare Gruppe P-Sp-X bedeutet.

8. Chirale Verbindungen nach mindestens einem der Ansprüche 1 bis 7, ausgewählt aus den folgenden Formeln

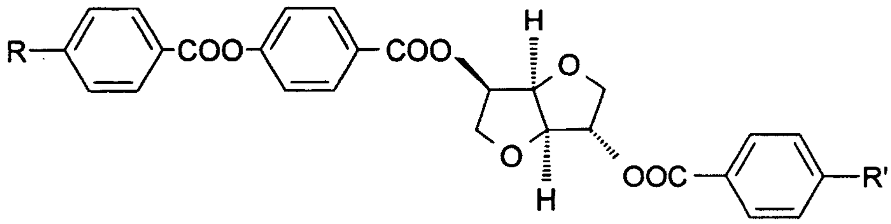

I-1

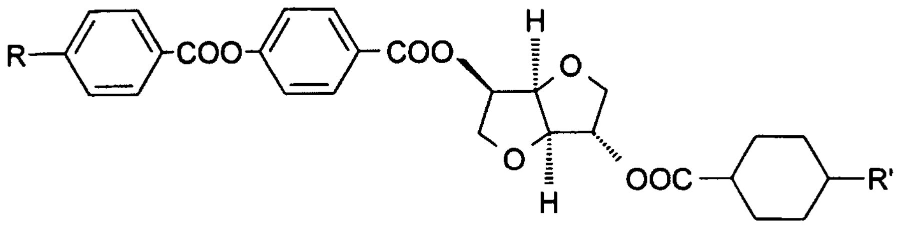

I-2

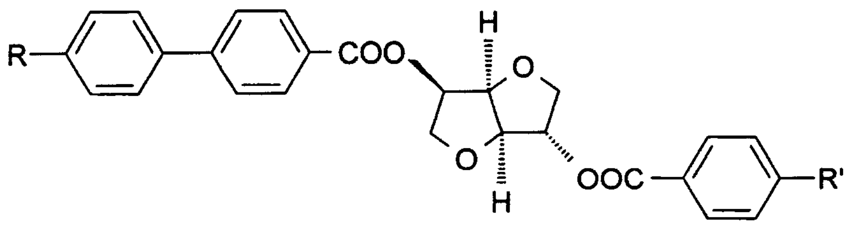

I-3

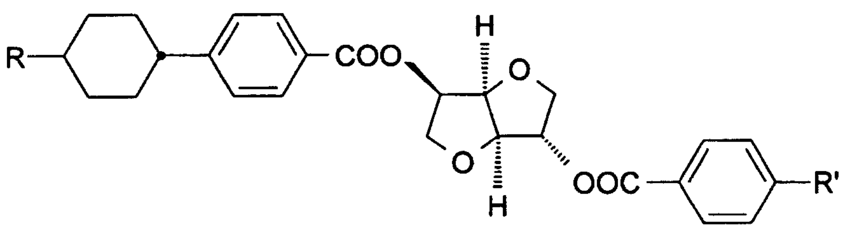

I-4

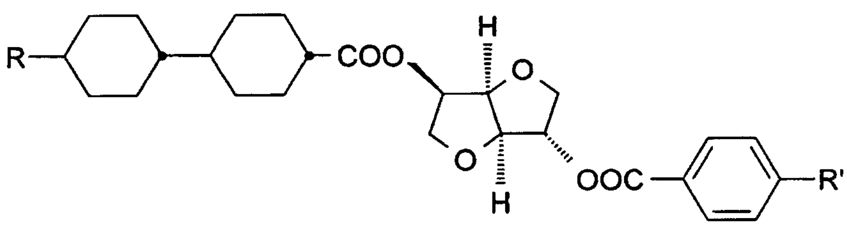

I-5

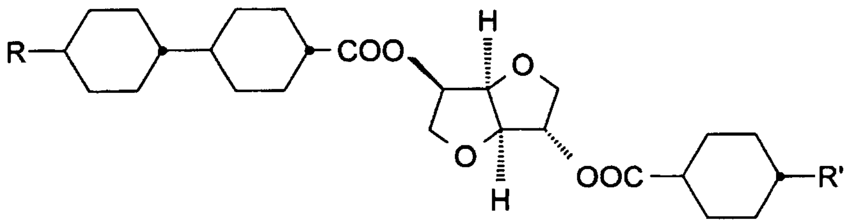

I-6

worin R und R' eine der Bedeutungen von $R^1$ in Anspruch 1 besitzen und die Phenylenringe auch durch 1, 2 oder 3 Gruppen L wie in Anspruch 1 definiert substituiert sein können.

**9.** Flüssigkristallmischung enthaltend mindestens eine chirale Verbindung nach mindestens einem der Ansprüche 1 bis 8.

**10.** Polymerisierbare Flüssigkristallmischung enthaltend mindestens eine Verbindung der Formel I nach mindestens einem der Ansprüche 1 bis 8 und mindestens eine polymerisierbare mesogene Verbindung, bei der es sich um die Verbindung der Formel I und/oder eine zusätzliche polymerisierbare mesogene Verbindung handeln kann.

**11.** Chirales lineares oder vernetztes Flüssigkristallpolymer erhältlich durch Polymerisieren einer Mischung nach Anspruch 10.

**12.** Verwendung einer chiralen Verbindung, einer chiralen Flüssigkristallmischung oder eines chiralen Polymers nach mindestens einem der Ansprüche 1 bis 10 in Flüssigkristallanzeigen, wie zum Beispiel STN-, TN-, AMD-TN-, Temperaturkompensations-, ferroelektrischen, Guest-Host-, Phasenwechselanzeigen oder Anzeigen mit oberflächenstabilisierter oder polymerstabilisierter cholesterischer Textur (SSCT, PSCT), in aktiven und passiven optischen Elementen wie Polarisatoren, Kompensatoren, Orientierungsschichten, Farbfiltern oder holographischen Elementen, in Klebstoffen, Kunstharzen mit anisotropen mechanischen Eigenschaften, kosmetischen oder pharmazeutischen Zusammensetzungen, z.B. als UV-Filter, Diagnostika, Flüssigkristallpigmenten, für dekorative und Sicherheitsanwendungen, in der nichtlinearen Optik, in der optischen Datenspeicherung oder als chirale Dotierstoffe.

**13.** Flüssigkristallanzeige enthaltend eine Flüssigkristallmischung nach Anspruch 9 oder 10.

**14.** Cholesterische Flüssigkristallmischung enthaltend eine chirale Komponente, die mindestens eine chirale Verbindung nach mindestens einem der Ansprüche 1 bis 8 enthält, und weiterhin enthaltend eine nematische Komponente, die eine oder mehrere nematische oder nematogene Verbindungen enthält.

**15.** Cholesterische oder SSCT-Anzeige enthaltend eine Mischung nach Anspruch 14.

**16.** Farbfilter, reflektierender Breitbandpolarisator, Strukturfolie oder Sicherheitskennzeichnung erhalten aus einer polymerisierbaren Mischung nach Anspruch 10.

## Revendications

**1.** Composés chiraux de la formule I

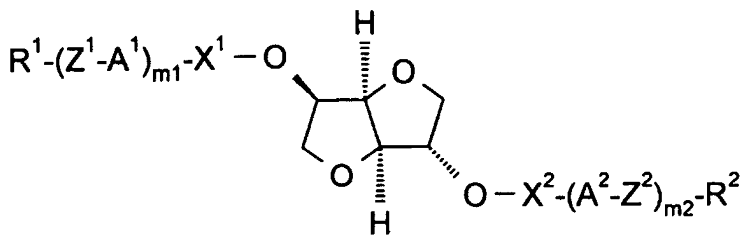

I

dans laquelle

$R^1$ et $R^2$ sont, indépendamment l'un de l'autre, F, Cl, Br, I, CN, SCN, $SF_5$, un alkyle à chaîne droite ou ramifié avec jusqu'à 30 atomes de C qui peut être non substitué, mono- ou polysubstitué par F, Cl, Br, I ou CN, étant également possible qu'un ou plusieurs groupes $CH_2$ non adjacents soient remplacés, dans chaque cas indépendamment les uns des autres, par -O-, -S-, -NH-, $-NR^0-$, -CO-, -COO-, -OCO-, -OCO-O-, -S-CO-, -CO-S-, -CH=CH- ou -C≡C- de telle sorte que des atomes de O et/ou de S ne soient pas liés directement les uns aux autres, ou P-Sp-X,

$R^0$ est H ou alkyle avec de 1 à 4 atomes de C,

P est un groupe polymérisable,

Sp est un groupe d'espaceur pour une liaison simple,

X est -O-, -S-, $-OCH_2-$, $-CH_2O-$, -CO-, -COO-, -OCO-, -OCO-O-, $-CO-NR^0-$, $-NR^0-CO-$, $-OCH_2-$, $-CH_2O-$, $-SCH_2-$, $-CH_2S-$, -CH=CH-COO-, -OOC-CH=CH- ou une liaison simple,

$X^1$ est -CO-, -OCO-, $-NR^0-CO-$, -CH=CH-CO-, $-CH_2-$, $-C_2H_4-$, $-CF_2-$ ou une liaison simple,

$X^Z$ est -CO-, -COO-, $-CO-NR^0-$, -CO-CH=CH-, $-CH_2-$, $-C_2H_4-$, $-CF_2-$ ou une liaison simple,

$Z^1$ et $Z^2$ sont, indépendamment l'un de l'autre, -O-, -S-, -CO-, -COO-, -OCO-, -O-COO-, $-CO-NR^0-$, $-NR^0-CO-$, $-OCH_2-$, $-CH_2O-$, $-SCH_2-$, $-CH_2S-$, $-CF_2O-$, $-OCF_2-$, $-CF_2S-$, $-SCF_2-$, $-CH_2CH_2-$, $-CF_2CH_2-$, $-CH_2CF_2-$, $-CF_2CF_2-$, -CH=N-, -N=CH-, -N=N-, -CH=CH-, -CF=CH-, -CH=CF-, -CF=CF-, -C≡C-, -CH=CH-COO-, -OCO-CH=CH- ou une liaison simple,

$A^1$ et $A^2$ sont, indépendamment l'un de l'autre, un groupe carboxylique ou hétérocyclique aliphatique ou aromatique avec jusqu'à 16 atomes de C qui peut également comprendre des anneaux fusionnés et qui peut être non substitué, mono- ou polysubstitué par L,

L est halogène ou un groupe cyano, nitro, alkyle, alkoxy, alkylcarbonyle ou alkoxycarbonyle avec de 1 à 7 atomes de C, où un ou plusieurs atomes de H peuvent être substitués par F ou Cl,

m1 est 1, 2 ou 3 ; et

m2 est 0, 1, 2 ou 3,

étant entendu que le nombre total d'anneaux fusionnés ou non fusionnés dans le groupe $(Z^1-A^1)_{m1}$ est supérieur au nombre dans le groupe $(A^2-Z^2)_{m2}$.

**2.** Composés chiraux selon la revendication 1, dans lesquels $A^1$ et $A^2$ sont choisis parmi 1,4-phénylène, où, en plus, un ou plusieurs groupes CH peuvent être remplacés par N, 1,4-cyclohexylène où, en plus, un ou deux groupes $CH_2$ non adjacents peuvent être remplacés par O et/ou S, 1,3-dioxolane-4,5-diyle, 1,4-cyclohexénylène et pipéridine-1,4-diyle, étant entendu qu'il possible que tous ces groupes soient non substitués, mono- ou polysubstitués par L comme défini selon la formule I, et où m1 > m2.

**3.** Composés chiraux selon la revendication 1 ou 2, dans lesquels m1 vaut 2 et m2 vaut 1.

**4.** Composés chiraux selon au moins l'une des revendications 1 à 3, dans lesquels $X^1$ et $X^2$ sont -CO-.

**5.** Composés chiraux selon au moins l'une des revendications 1 à 4, dans lesquels $A^1$ et$A^2$ sont choisis parmi trans-1,4-cyclohexylène et 1,4-phénylène, le second étant non substitué ou substitué par jusqu'à 4 groupes L.

**6.** Composés chiraux selon au moins l'une des revendications 1 à 5, dans lesquels $Z^1$ et $Z^2$ sont -COO-, -OCO ou une liaison simple.

**7.** Composés chiraux selon au moins l'une des revendications 1 à 6, dans lesquels $R^1$ et/ou $R^2$ est un groupe polymérisable P-Sp-X.

**8.** Composés chiraux selon au moins l'une des revendications 1 à 7, choisis parmi les formules qui suivent

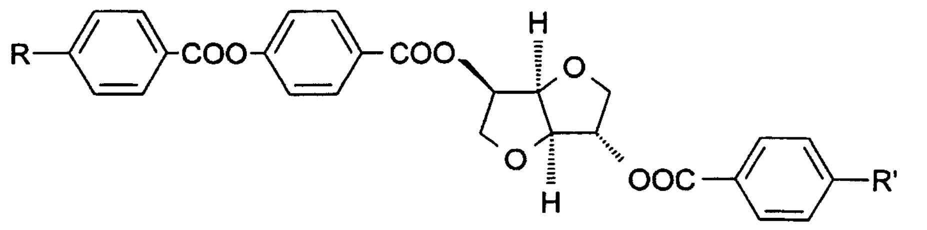

I-1

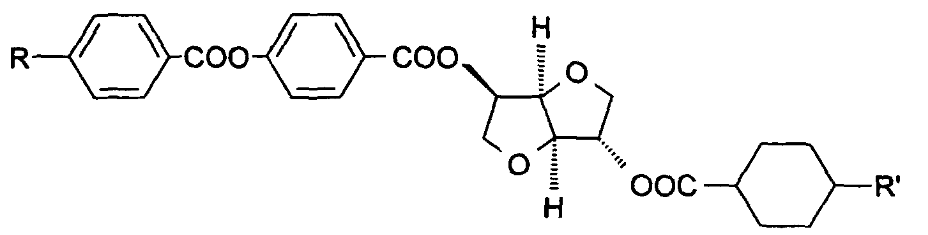

I-2

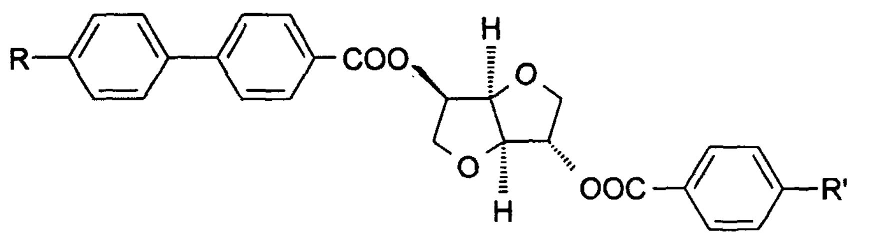

I-3

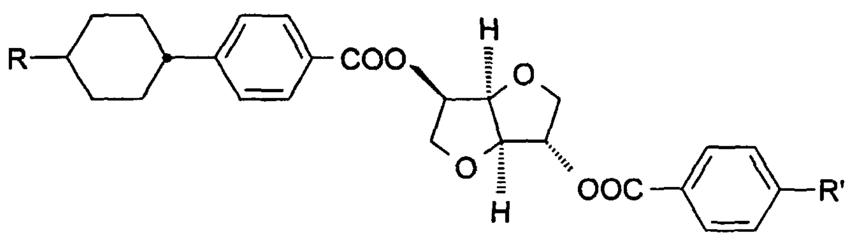

I-4

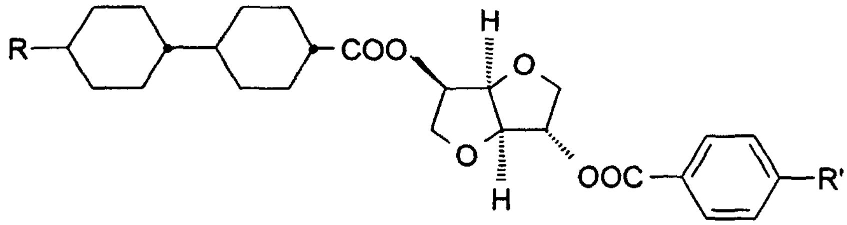

I-5

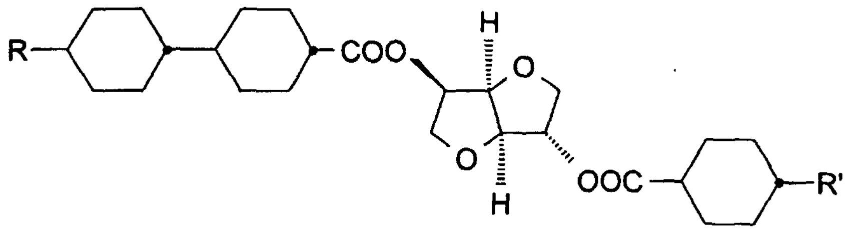

I-6

dans lesquels R et R' présentent l'une des significations de $R^1$ selon la revendication 1 et les anneaux phénylène peuvent également être substitués par 1, 2 ou 3 groupes L comme défini selon la revendication 1.

**9.** Mélange de cristaux liquides comprenant au moins un composé chiral selon au moins l'une des revendications 1 à 8.

**10.** Mélange de cristaux liquides polymérisables comprenant au moins un composé de la formule I selon au moins l'une des revendications 1 à 8 et au moins un composé mésogène polymérisable, qui peut être ledit composé de la formule I et/ou un composé mésogène polymérisable additionnel.

**11.** Polymère de cristaux liquides linéaire chiral ou réticulé pouvant être obtenu en polymérisant un mélange selon la revendication 10.

**12.** Utilisation d'un composé chiral, d'un mélange de cristaux liquides ou d'un polymère selon au moins l'une des revendications 1 à 10 dans des affichages à cristaux liquides, tels que par exemple des affichages STN, TN, AMD-TN, à compensation de température, ferroélectriques, invité-hôte, à changement de phase ou à texture cholestérique stabilisée en surface ou stabilisée par polymère (SSCT, PSCT), dans des éléments optiques actifs et passifs, tels que des polariseurs, des compensateurs, des couches d'alignement, des filtres couleur ou des éléments holographiques, dans des adhésifs, des résines synthétiques avec des propriétés mécaniques anisotropes, des compositions cosmétiques ou pharmaceutiques, par exemple telles que des filtres UV, des moyens de diagnostic, des pigments de cristaux liquides, pour des applications de décoration et de sécurité, dans des optiques non linéaires, dans une mémoire d'information optique ou en tant que dopants chiraux.

**13.** Affichage à cristaux liquides comprenant un mélange de cristaux liquides selon la revendication 9 ou 10.

**14.** Mélange de cristaux liquides cholestériques comprenant un composant chiral qui contient au moins un composé chiral selon au moins l'une des revendications 1 à 8, et comprenant en outre un composant nématique qui contient un ou plusieurs composés nématiques ou nématogènes.

**15.** Affichage cholestérique ou SSCT comprenant un mélange selon la revendication 14.

**16.** Filtre couleur, polariseur à réflexion large bande, film conformé ou marquage de sécurité obtenu à partir d'un mélange polymérisable selon la revendication 10.